# DEMANDE DE BREVET EUROPEEN

(11) **EP 0 575 274 A1**
(43) Date de publication de la demande: **22.12.1993**
(21) Numéro de dépôt: 93430008.8
(22) Date de dépôt: 15.06.1993
(51) Int. Cl.: A61C 1/00, A61B 17/36

(54) **Appareil laser pour traitements médicaux et dentaires**

(30) Priorité: 16.06.1992 US 899288
(71) Demandeur: LASER MEDICAL TECHNOLOGY, Inc., San Clemente, California 92672 (US)
(72) Inventeur: Levy, Guy, Tustin, California 92680 (US)
(74) Mandataire: Marek, Pierre

(57) **Abrégé**

Appareil de traitement médical, caractérisé en ce qu'il comprend :
- des moyens laser comprenant une première (6) et une seconde (8) source d'émission de rayonnements laser, aptes à produire, chacune, un rayonnement laser d'une longueur d'onde et d'un niveau de puissance respectivement différents, capables de couper un tissu organique donné ;
- des moyens (32, 46) d'application du rayonnement laser pour diriger ledit rayonnement laser sur le tissu corporel à traiter ;
- des moyens de conduite ou canalisation du rayonnement laser, comportant au moins une fibre optique (30 ; 42, 44) connectée pour conduire le rayonnement laser depuis les sources d'émission (6, 8) jusqu'auxdits moyens d'application (32, 46) ; et
- des moyens d'alimentation en fluide de refroidissement (18), pour diriger un fluide de refroidissement contenant de l'air et de l'eau vers le tissu corporel sur lequel est appliqué le rayonnement laser.

## Description

La présente invention concerne un appareil constituant une source de rayonnement laser plus particulièrement utilisable pour des traitements médicaux et dentaires.

L'émission de rayonnement laser est couramment utilisée en médecine et en dentisterie, pour l'exécution d'une grande variété de procédures incluant des opérations qui nécessitent la coupe et la vaporisation de tissus mous. Plus précisément, l'utilisation des dispositifs connus sous le nom de scalpels laser, ou de bistouris laser, en lieu et place des scalpels traditionnels, est maintenant bien acceptée.

On sait également que le rayonnement laser peut être utilisé pour favoriser la guérison et, entre autres, pour cautériser, coaguler et stériliser.

Par ailleurs, des recherches importantes ont démontré la capacité qu'a un rayonnement laser de longueur d'onde et de densité d'énergie appropriées, de couper des tissus durs, y compris l'os, l'émail, la dentine et le cément, aussi bien que les tissus durs déminéralisés, tel que le tissu dentaire carié. Dans les applications dentaires, les tissus mous qui peuvent être coupés comprennent la matière gingivale, le tissu nerveux et la pulpe. Le rayonnement laser peut également être utilisé pour couper le tartre, les plaques ou les calculs qui se forment sur les surfaces des dents, tout comme les matières similaires qui s'accumulent dans les passages corporels, y compris les vaisseaux sanguins et les voies urinaires.

Le terme "couper", tel qu'il est utilisé dans l'exposé qui suit, englobe des mécanismes tels que la vaporisation qui peut être produite par photoablation, ou par photodisruption.

L'effet d'un faisceau laser sur un tissu particulier quelconque dépend essentiellement de la longueur d'onde et du niveau de puissance du rayonnement, ainsi que de la forme sous laquelle le rayonnement est émis, c'est-à-dire en onde continue, ou en impulsions. Dès lors, il est de pratique courante, lorsqu'on cherche à exécuter une opération particulière, que ce soit dans un but clinique ou en rapport avec la recherche scientifique, de rechercher le type de laser dont la longueur d'onde offre le meilleur rendement pour cette opération et, ensuite, d'essayer de trouver les valeurs optimales pour le niveau de puissance et, si une émission de radiations par impulsions semble préférable, les valeurs optimales pour la durée et la fréquence de récurrence des impulsions. L'énergie fournie par chacune de ces impulsions sera le produit de la puissance de sortie du laser et de la durée des impulsions. La densité d'énergie, avec laquelle chaque impulsion est appliquée sur le tissu en cours de traitement, dépendra du diamètre de la tache focale ou surface d'exposition du rayonnement sur le tissu, et constitue un paramètre important pour déterminer l'effet d'un tel rayonnement laser.

Dans les opérations du genre décrit précédemment, la possibilité d'appliquer le rayonnement en un emplacement voulu revêt une importance capitale, et il est connu d'appliquer le rayonnement laser au moyen d'une pièce à main qui peut être manipulée et orientée facilement par le médecin ou le dentiste. Or, comme les lasers, en particulier ceux qui doivent produire les niveaux de puissance requis pour de tels traitements médicaux, sont eux-mêmes des dispositifs relativement encombrants, une pièce à main ne peut être utilisée que s'il existe un moyen permettant d'amener le faisceau laser du laser proprement dit à ladite pièce à main. Les fibres optiques constituent une solution logique et séduisante à ce problème. Cependant, le rayonnement produit par un certain nombre de lasers couramment utilisés à ce jour ne peut être transmis par l'intermédiaire de fibres optiques. C'est le cas, par exemple, pour le rayonnement produit par le laser au CO₂.

Par ailleurs, on sait que les longueurs d'onde de rayonnement allant d'environ 0,5µ , au moins, à environ 3µ , peuvent être transmises efficacement par le biais des fibres optiques traditionnelles, au moins à des niveaux de puissances relativement bas.

Les rayonnements laser d'une longueur d'onde inférieure à 3µ comprennent ceux produits par le laser YAG:Nd (longueur d'onde fondamentale 1,06µ ) et par le laser YAG:Er (longueur d'onde fondamentale 2,94µ ). Ces deux formes d'émissions laser sont capables de couper différents types de tissus, bien que chacun fasse appel à un mécanisme quelque peu différent pour ce faire. Cette différence tient, d'une part, à ce que l'eau présente un très faible coefficient d'absorption du rayonnement à la longueur d'onde de 1,06µ , et un coefficient d'absorption relativement élevé pour un rayonnement à la longueur d'onde de 2,94µ .

Les documents US-A-4.931.053 et US-A-4.951.663 divulguent un appareil comprenant deux lasers qui produisent des faisceaux laser alignés sur un axe de sortie commun. Le rayonnement laser est choisi de manière à éviter d'induire une photocoagulation, une détérioration photonoptique du tissu, une photovolatisation ou une décomposition par photo-ablation des tissus ou cellules du corps atteint.

Suivant le document US-A-4.931.053, le rayonnement est choisi de manière à porter la croissance vasculaire ou similaire, au-delà du seuil qui peut être atteint par un seul laser.

Suivant le document US-A-4.951.663, le rayonnement laser est choisi de manière à créer un système biomédical de stérilisation susceptible de détruire des micro-organismes, tant dans le derme que dans l'épiderme.

Le document US-A-4.925.523 décrit un appareil dans lequel des faisceaux laser aux longueurs d'onde de 193 nm et de 308 nm, sont alignés sur un axe de sortie commun, et sont appliqués sur une pièce de travail, en vue de produire un effet d'ablation renforcé. Le rayonnement de 193 nm excite les molécules de la matière de la pièce traitée, afin d'amener certaines de ces molécules à se sublimer, et d'autres à passer à l'état de triplet, sans ablation. La longueur d'onde de 308 nm produit l'ablation des molécules qui ont été mises à l'état de triplet. La longueur d'onde de 308 nm ne peut produire par elle-même l'ablation de molécules qui n'ont pas été excitées. Ce Brevet mentionne d'autres paires de longueurs d'onde, décrit l'effet du rayonnement sur divers matériaux plastiques, et avance l'hypothèse que la méthode peut être mise en oeuvre pour attaquer des substrats organiques, y compris les tissus, les os et les dents.

Le document US-A 4.408.602, divulgue un système à deux sources de coupe laser, dont chacune agit sur différents types de tissus, des moyens étant prévus pour passer d'une source à l'autre.

La présente invention a pour but de créer un appareil permettant de perfectionner le traitement qui peut être exécuté au moyen d'un rayonnement laser, et d'élargir la variété de traitements susceptibles d'être exécutés avec sécurité et efficacité par l'utilisation d'un faisceau laser.

L'invention a pour autre but de combiner les avantages qu'offre la possibilité d'utiliser une pluralité de longueurs d'onde différentes, dont chacune peut produire individuellement un effet de coupe d'un tissu organique, par vaporisation, en particulier d'une manière qui remédie aux insuffisances de l'une ou de plusieurs de ces longueurs d'onde.

L'invention a pour autre but la réalisation d'un appareil permettant d'obtenir un niveau de contrôle plus élevé des procédures médicales et dentaires qui mettent en oeuvre un rayonnement laser.

Suivant l'invention, les buts exposés ci-dessus et d'autres encore sont atteints grâce à un appareil de traitement médical comprenant des moyens laser qui comportent une première et une seconde source de rayonnement laser, chacune destinée à produire un rayonnement ou faisceau laser d'une longueur d'onde respective différente, capable de couper un tissu organique ; des moyens d'application du rayonnement laser, pour diriger ledit rayonnement sur le tissu corporel à traiter ; des moyens de conduite ou canalisation du rayonnement laser, comportant au moins une fibre optique, connectée pour conduire le rayonnement depuis les sources d'émission jusqu'aux moyens d'application ; et des moyens d'alimentation en fluide de refroidissement, pour diriger un fluide de refroidissement sur le tissu corporel sur lequel est dirigé le rayonnement laser.

Les sources d'émission du rayonnement laser sont, de préférence, un laser YAG-Nd et un laser YAG-Er.

La figure 1 est une vue en coupe simplifiée, à caractère schématique, montrant les principaux composants d'une unité ou appareil laser selon l'invention.

Les figures 2 et 3 sont des vues simplifiées, à caractère schématique, illustrant deux agencements pour diriger le rayonnement laser produit par les moyens laser de la figure 1.

Selon un mode de réalisation préféré de l'invention, l'appareil laser est un laser combiné YAG-Nd et YAG:Er. Dans la suite du présent exposé, ces dispositifs seront appelés "laser Nd" et "laser Er", dans un but de concision.

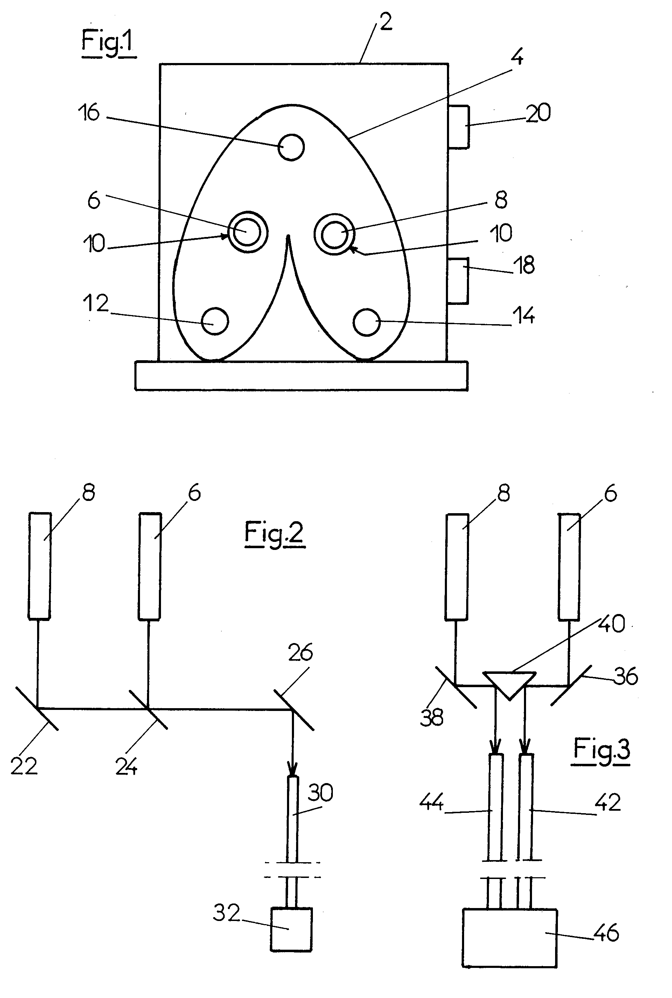

L'appareil illustré par la figure 1 comprend un logement 2 qui renferme un réflecteur optique 4 comprenant un laser constitué d'un barreau laser YAG 6 dopé au Nd, et un laser constitué d'un barreau laser YAG 8 dopé à l'Er. Chacun des barreaux 6 et 8 est entouré d'un filtre optique 10 adéquat.

La zone délimitée par le réflecteur 4 renferme également trois lampes à éclairs 12, 14 et 16.

Les lampes 12, 14 et 16 émettent un rayonnement lumineux qui produit le pompage optique des lasers Nd et Er.

Dans le mode de réalisation illustré sur la figure 1, le réflecteur 4 a une forme en double ellipse approchant celle de deux ellipses qui se chevauchent partiellement, le réflecteur 4 étant configuré de manière à concentrer la quasi totalité de la lumière produite par la lampe 12 dans le barreau 6, et à concentrer la quasi totalité de la lumière produite par la lampe 14 dans le barreau 8, tandis que la lumière produite par la lampe 16 est répartie de manière sensiblement égale entre les barreaux 6 et 8.

L'intérieur du logement 2 est, de préférence, refroidi à l'eau, la circulation de l'eau entre les parois du logement 2 et une baisse de la chaleur étant obtenues par l'intermédiaire d'une unité de couplage 18. La puissance électrique nécessaire pour les lampes 12, 14 et 16 est fournie dans le logement 2 par l'intermédiaire d'une unité de connexion 20. Avec des réflecteurs adéquats (non représentés) disposés, de manière classique, aux extrémités des barreaux 6 et 8, l'émission de lumière à partir des lampes 12 et 14 et/ou de la lampe 16 provoque la génération d'un rayonnement laser et son émission, par l'intermédiaire d'une portion transparente du réflecteur, à une extrémité de chaque barreau.

La puissance d'émission produite par chaque barreau 6, 8 sera fonction de l'intensité et de la longueur d'onde de la lumière fournie à chacun des barreaux par les lampes 12, 14 et 16. Par conséquent, la relation entre les niveaux de puissance du rayonnement émanant des deux barreaux 6 et 8 peut être ajustée en faisant varier la tension appliquée à chacune des lampes 12, 14 et 16. Dans ce cas, la variation de la tension à travers la lampe 16 aura un effet proportionnel sur la puissance de sortie des deux barreaux 6, 8.

De plus, des impulsions de rayonnement laser peuvent être produites en faisant fonctionner l'une ou l'autre des lampes 12 et 14 en impulsions, c'est-à-dire que la durée et la fréquence de récurrence des impulsions produites par chaque barreau 6, 8 correspondront à la durée et à la fréquence de récurrence, respectivement, des impulsions de tension de régime appliquée à l'une ou l'autre des lampes 12 et 14. La commande du fonctionnement des lampes 12 et 14 peut s'effectuer par un système d'alimentation en énergie commandé par microprocesseur.

Selon une caractéristique particulière de l'invention, les lampes 12, 14 et 16 peuvent être remplies d'un mélange spécial de krypton et de xénon, pour produire un pompage optimal du milieu laser dans les barreaux 6 et 8. Ce mélange peut varier dans une large gamme de proportions. Une amélioration concernant l'utilisation d'un seul ou de l'autre des ingrédients deviendra apparente si chaque ingrédient est présent dans le mélange dans une concentration supérieure à 2 % en poids.

Lorsque l'émission laser Nd et/ou Er prend la forme d'un train d'impulsions, le niveau de puissance du rayonnement laser émis à partir de chaque barreau laser 6, 8 peut être réglé individuellement, tout comme peut l'être la synchronisation d'impulsions, durée et taux de fréquence des impulsions, en appliquant individuellement des tensions de commande de fonctionnement à chacune des lampes 12 et 14 et également à la lampe 16 à un niveau tel que sa puissance de sortie ne puisse, par elle-même, amener le barreau 6 ou 8 à émettre un rayonnement.

Deux agencements possibles pour conduire ou canaliser le rayonnement jusqu'à une pièce à main sont représentés sur les figures 2 et 3.

Selon le mode de réalisation montré sur la figure 2, le rayonnement en provenance du laser Er 8 est réfléchi par un premier miroir 22, passe à travers un deuxième miroir 24, et est réfléchi par un troisième miroir 26 sur l'extrémité d'entrée d'une fibre de transmission 30. Le rayonnement du laser Nd 6 est en premier lieu réfléchi par le deuxième miroir 24, et est ensuite réfléchi par le troisième miroir 26 sur l'extrémité d'entrée de la fibre 30. Selon les principes connus de l'état de la technique, les miroirs 22, 24 et 26 peuvent être des miroirs dichroiques, construits chacun pour réfléchir une longueur d'onde de rayonnement choisie et/ou transmettre une longueur d'onde choisie. Plus particulièrement, le miroir 22 est conçu de manière à réfléchir, dans la mesure la plus large possible, le rayonnement Er à une longueur d'onde de 2,94 microns, le miroir 24 est conçu pour être hautement transmissif vis-à-vis du rayonnement Er, tout en ayant la plus haute réflectivité possible à l'égard du rayonnement Nd à une longueur d'onde de 1,06 micron, et le miroir 26 est conçu pour avoir la plus haute réflectance possible à l'égard des deux longueurs d'onde de 1,06 et 2,94 microns.

La fibre 30 s'étend de la région adjacente au miroir 26 jusqu'à une pièce à main 32 adéquate.

Suivant le mode de réalisation représenté sur la figure 3, les rayonnements sortant des barreaux 6 et 8 sont réfléchis par des miroirs respectifs 36 et 38 vers un réflecteur 40, qui peut être un combinateur laser à cristal ouvert non-linéaire ou à "cellule di", permettant de diriger le rayonnement émis à partir de chaque barreau vers les entrées de l'une des deux fibres optiques 42 et 44 respectives, qui s'étendent en direction d'une pièce à main 46 appropriée à laquelle elles sont connectées.

L'utilisation de deux fibres optiques permet de fournir un niveau de puissance totale plus élevé à la pièce à main 46, tout en permettant que les fibres 42 et 44 soient suffisamment fines pour avoir un degré de flexibilité élevé. La pièce à main 46 peut comporter des éléments conventionnels de combinaison des rayonnements, pour orienter les rayonnements venant des deux sources selon un axe commun.

Les appareils illustrés sur le dessin et précédemment décrits peuvent être employés pour émettre un rayonnement Nd, un rayonnement Er, ou une combinaison de ces deux types de rayonnement, avec n'importe quelle combinaison voulue des niveaux de puissance, des durées des impulsions et des fréquences de récurrence des impulsions. Il en résulte qu'on peut utiliser un seul appareil pour exécuter une grande variété d'opérations, et dépasser grandement les possibilités offertes par les appareils de l'art antérieur, en exécutant des opérations variées par la combinaison des deux types de rayonnements.

On décrit ci-après quelques exemples des avantages qui peuvent être obtenus par l'utilisation d'un appareil conforme à l'invention, en référence au mode de réalisation spécifique précédemment décrit, qui comprend une source d'émission laser Nd et une source d'émission laser Er.

Des recherches antérieures ont démontré que lorsqu'il est utilisé avec un système de refroidissement, le rayonnement Nd peut agir à la fois sur les tissus durs et les tissus mous. Les tissus durs peuvent notamment être coupés, ou vaporisés, avec un rayonnement de fréquence fondamentale de 1,06 micron, alors que les tissus mous peuvent être coupés plus efficacement avec un rayonnement de fréquence doublée à une longueur d'onde de 0,532 micron. Ces deux longueurs d'onde ne sont pratiquement pas absorbées par l'eau et agissent directement sur les constituants tissulaires non-aqueux. L'apport d'un brouillard de refroidissement aqueux dans la région irradiée supprime la chaleur générée par l'émission laser et a pour but de réduire le plus possible la surface de l'enveloppe endommagée autour de la région tissulaire qui a été coupée ou vaporisée.

Le laser Er, dont l'utilisation est apparue plus récemment dans ce domaine, produit un rayonnement à une longueur d'onde qui est fortement absorbée par l'eau, et très fortement absorbée par l'hydroxy-apatite. Dès lors, le laser Er peut couper efficacement et les tissus mous, et les tissus durs. Lorsqu'un tel laser est utilisé sans refroidir simultanément le tissu irradié, les résultats histologiques ne sont pas bons, en ce sens que la guérison n'intervient que relativement lentement, en raison de la création d'une enveloppe endommagée importante et d'une légère carbonisation autour de la région qui a été vaporisée ou coupée.

Le laser Er s'avère capable de couper ou de vaporiser l'émail dentaire avec une grande efficacité, car ce tissu contient une faible quantité d'eau, entre 2 et 3 %, ce qui fait que l'énergie du rayonnement laser est efficacement absorbée par l'hydroxy-apatite. En fait, il est possible d'obtenir un plasma avec photodisruption, avec des impulsions de rayonnement d'un niveau de puissance moyen peu élevé, qui peut être compris entre 3 et 6 watts. La durée des impulsions peut être de l'ordre de 100 µs à 1 ms. La fréquence de récurrence ou taux de répétition des impulsions peut être de l'ordre de 30-50 Hz, et le rayonnement peut être focalisé, sur la surface à couper, en une tache d'une taille de 150-600µ . D'autres valeurs peuvent toutefois s'avérer adéquates pour ces paramètres.

On a cependant découvert que le rayonnement émanant du laser Er peut avoir des effets secondaires indésirables sur l'émail, impliquant l'apparition de fissures lorsque ce rayonnement est appliqué sans projection simultanée d'un liquide de refroidissement. Il a été prouvé que lorsque le refroidissement est produit par la projection d'un jet ou d'un brouillard de liquide de refroidissement, l'apparition de fissures était évitée. Or, le rayonnement laser Er étant absorbé dans une large mesure par l'eau, l'efficacité de ce laser se trouve considérablement réduite en la présence d'un jet ou d'un brouillard de refroidissement, ce qui signifie que l'énergie des impulsions de rayonnement doit être accrue, pour pouvoir réaliser une coupe efficace.

Le laser Er est également capable de couper la dentine et l'os, mais avec moins d'efficacité que le laser Nd, et il a tendance à engendrer une carbonisation, en raison du fait que ces tissus renferment une grande quantité d'eau, entre 20 et 30 %, qui absorbe l'énergie de rayonnement du laser Er et se trouve chauffée par cette dernière. Ce rayonnement étant absorbé dans une large mesure par l'eau, les impulsions du laser doivent avoir un niveau d'énergie élevé. Il s'ensuit que les effets thermiques deviennent plus importants, ce qui se traduit par la création d'une enveloppe endommagée relativement importante autour du tissu coupé, ce qui ralentit le processus de guérison.

De plus, il est plus difficile de conduire ou canaliser le rayonnement Er par l'intermédiaire de fibres optiques conventionnelles. Plus précisément, le rayonnement du laser Er acheminé par de telles fibres optiques ne peut avoir un niveau de puissance moyen de plus de 3 ou 4 watts, environ, en raison de la plus grande longueur d'onde du rayonnement du laser Er.

D'autre part, le rayonnement du laser Nd est absorbé dans une large mesure par l'hydroxy-apatite et n'est pratiquement pas absorbé par l'eau, c'est-à-dire que l'eau est transparente à la longueur d'onde de ce rayonnement. Aussi, le rayonnement du laser Nd est très efficace pour couper les tissus mous, comme les tissus durs. De plus, le rayonnement du laser Nd peut être facilement fourni à des niveaux de puissance élevés par des fibres optiques conventionnelles à la silice, même par des fibres ayant un diamètre de l'ordre de 100 à 150 microns, qui peuvent être effectivement utilisées pour l'élargissement des canaux radiculaires ou mise en forme canalaire.

Sur un tissu dur, le rayonnement du laser Nd peut couper efficacement l'os, la dentine, le cément ou l'émail, et une photodisruption peut être obtenue même avec des niveaux de puissance moyens peu élevés, de l'ordre de 6 watts, et même avec des impulsions de longue durée, car la densité d'énergie appliquée au tissu peut être portée à un niveau suffisamment élevé pour produire un effet de photodisruption qui engendre une rupture acoustique évitant la transmission directe de chaleur au tissu. Lorsqu'il est combiné à l'apport d'un jet de fluide de refroidissement, de préférence un brouillard à base d'air et d'eau, cet effet de photodisruption ne produit qu'une enveloppe endommagée de très petite taille, et permet au processus de guérison d'intervenir rapidement. L'enveloppe endommagée est la région qui borde le vide ou le trou produit par la vaporisation du tissu, et dans lequel on peut observer un dommage au tissu à la suite de son exposition au rayonnement laser. De plus, le rendement, avec lequel l'os et la dentine peuvent être coupés avec un rayonnement laser Nd, est très bon. Cependant, on a découvert qu'en ce qui concerne l'émail, ses cristaux réfléchissent et dispersent une partie de l'énergie du rayonnement.

De meilleurs résultats sont obtenus en combinant le rayonnement laser Er et le rayonnement laser Nd pour des opérations de coupe. Lorsque l'appareil selon l'invention est utilisé pour couper de l'émail en présence d'un brouillard d'eau de refroidissement, l'efficacité de coupe du rayonnement laser Er se trouve réduite, mais le couplage entre les rayonnements Er et Nd rehausse l'efficacité de coupe du rayonnement Nd, car la photodisruption produite par le rayonnement Er augmente, même dans des domaines de basses puissances de l'ordre de 3-4 watts, l'absorption du rayonnement Nd par l'émail qui aura été transformé par la photodisruption. Effectivement, une telle photodisruption modifie ou détruit la structure cristalline de l'émail atteint. Il s'ensuit que la dispersion du rayonnement à la longueur d'onde de 1,06µ , qui serait produite par la structure cristalline, est substantiellement réduite, ce qui fait que l'absorption du rayonnement à 1,06µ est sensiblement renforcée. Dès lors, l'efficacité de la coupe est rehaussée, sans que le rayonnement Er doive avoir un niveau de puissance élevé. Cela signifie que les deux types de rayonnements peuvent être conduits par une fibre conventionnelle à la silice, dopée au fluorure, et présentant un diamètre de 600µ ou moins, sans que la fibre soit brûlée ou autrement endommagée.

De plus, il peut être fait usage de fibres jetables et de fibres présentant une extrémité distale conique, les fibres s'effilant à ladite extrémité, par exemple, d'un diamètre de 600µ à un diamètre de 100 à 200µ .

En conséquence de l'effet décrit ci-dessus, un rayonnement combiné conforme à l'invention peut couper efficacement l'émail, la dentine, le cément et l'os.

Dans le cas d'un tissu mou, le rayonnement laser Er produira une coupe peu profonde, superficielle, et le rayonnement laser à Nd approfondira la coupure, car il n'est absorbé que faiblement par l'eau. Des résultats satisfaisants peuvent être obtenus par un rayonnement laser Nd à une longueur d'onde de 1,06µ, de sorte que les modes de réalisation de l'invention peuvent être mis en oeuvre sans utilisation d'éléments de doublage de la fréquence, requis jusqu'à présent, pour donner au rayonnement laser Nd une longueur d'onde de 0,532µ .

Selon des modes de réalisation préférentiels de l'invention, le laser Er peut débiter une puissance de sortie à un niveau moyen de 3-6 watts, et le laser à Nd peut débiter une puissance de sortie à un niveau moyen de 1-15 watts. Pour couper de l'os, la puissance de sortie moyenne du laser Nd peut aller jusqu'à 40 watts.

Pour chacun des lasers 6, 8, la durée des impulsions peut, par exemple, être comprise entre 100 µs et plusieurs ms, de préférence 800 µs. La dimension de la tache du rayonnement peut être de 150-600 µ.

La fréquence de récurrence ou taux de répétition pour chaque longueur d'onde est, de préférence, de 50 Hz.

Les types de tissus mous qui peuvent être coupés comprennent les matières gingivales, pulpaires et radiculaires, et les tissus mous que l'on rencontre en chirurgie générale.

Les types de tissus durs qui peuvent être coupés comprennent l'émail sain ou carié, la dentine, le cément et l'os, en dentisterie, par exemple pour exécuter des apico-ectomies, ainsi qu'en traumatologie, en orthopédie et en médecine vétérinaire.

Pour les traitements endodontiques, le rayonnement selon l'invention peut être fourni par une fibre optique mince, pour donner accès à la chambre pulpaire, enlever la pulpe, préparer un canal radiculaire, stériliser la paroi du canal et pour nettoyer et former le canal.

A des niveaux d'énergie peu élevés, le rayonnement laser peut être utilisé pour désensibiliser le tissu dentaire, en particulier la structure radiculaire.

Le rayonnement peut aussi être utilisé pour les traitements périodontiques, pour stériliser une poche, couper la matiére gingivale et enlever les calculs et le tartre.

Le rayonnement peut encore être utilisé pour souder ou faire adhérer une préparation contenant de l'hydroxy-apatite à un matériau dentaire ou osseux.

Lorsqu'un tel rayonnement est appliqué sur un tissu, il demeure essentiel qu'un jet ou un brouillard contenant un liquide de refroidissement, de préférence un mélange d'air et d'eau, soit appliqué simultanément sur la région irradiée.

Un faisceau combiné de rayonnement laser Er et de rayonnement laser Nd peut également améliorer l'efficacité de la coupe, par vaporisation, de matériaux inorganiques, tels que l'acier. Dans ce cas, la photodisruption produite par le rayonnement laser Nd est améliorée par le rayonnement laser Er.

L'appareil selon l'invention peut égalemnt être utilisé pour opérer l'hémostase, ou l'arrêt des hémorragies, dans les blessures et les incisions. Cette opération demande des niveaux de densité d'énergie plus bas que ceux qui sont nécessaires pour produire une action de coupe, et peut donc être exécutée avec un faisceau laser formé pour avoir une tache plus grande que celle qui est nécessaire pour couper. Le rayonnement laser Er réalisera une hémostase superficielle, et le rayonnement laser Nd à 1,06µ opèrera une hémostase plus profonde. Si un rayonnement laser Nd à 0,532µ était disponible, il opèrerait une hémostase plus superficielle ; cette longueur d'onde est bien absorbée par un pigment rouge, comme celui qui est présent dans l'hémoglobine. Alors que les modes de réalisation préférés de l'invention comprennent, comme indiqué précédemment, un laser Er et un laser Nd, on peut employer d'autres lasers capables de créer un type de synergie semblable à ceux décrits ci-dessus. Par exemple, le laser Nd pourrait être remplacé par un laser à alexandrite, qui produit un rayonnement à une longueur d'onde de 0,730µ à 0,790µ . Un rayonnement dans cette gamme de longueur d'onde est substantiellement absorbé par l'hémoglobine et l'hydroxy-apatite, modérément ou moyennement absorbé par l'eau, et dispersé par l'émail. Pour des traitements appliqués aux tissus mous, tels que coupe et hémostase, ses effets se situent entre ceux produits par le rayonnement laser Nd à 0,532µ et 1.06µ .

Bien que la description qui précède se référe à des modes de réalisation particuliers de la présente invention, on comprendra que de nombreuses modifications peuvent être faites sans sortir de l'esprit de l'invention. Les revendications annexées sont réputées couvrir toute modification de ce genre, qui tomberait dans le cadre exact et l'esprit de la présente invention.

Les modes de réalisation présentement décrits doivent donc être considérés à tous égards comme illustratifs, et non limitatifs, le cadre de l'invention étant défini par les revendications annexées, plutôt que par la description qui précède, et tous les changements qui tombent sous le sens et dans le domaine d'équivalence des revendications sont donc réputés compris dans ces dernières.

## Revendications

**1. -** Appareil de traitement médical, caractérisé en ce qu'il comprend :
- des moyens laser comprenant une première (6) et une seconde (8) source d'émission de rayonnements laser, aptes à produire, chacune, un rayonnement laser d'une longueur d'onde et d'un niveau de puissance respectivement différents, capables de couper un tissu organique donné ;
- des moyens (32, 46) d'application du rayonnement laser pour diriger ledit rayonnement laser sur le tissu corporel à traiter ;
- des moyens de conduite ou canalisation du rayonnement laser, comportant au moins une fibre optique (30 ; 42, 44) connectée pour conduire le rayonnement laser depuis les sources d'émission (6, 8) jusqu'auxdits moyens d'application (32, 46) ; et
- des moyens d'alimentation en fluide de refroidissement (18), pour diriger un fluide de refroidissement contenant de l'air et de l'eau vers le tissu corporel sur lequel est appliqué le rayonnement laser.

**2. -** Appareil suivant la revendication 1, caractérisé en ce que lesdits moyens laser précités comprennent des moyens de commande du faisceau laser, pour appliquer le rayonnement émis par chacune desdites sources de rayonnement laser (6, 8), sous forme de train d'impulsions à une fréquence de récurrence ou taux de répétition sélectionnée, chaque impulsion ayant une durée sélectionnée.

**3. -** Appareil suivant la revendication 2, caractérisé en ce que chacune desdites sources de rayonnement comprend un corps ou matériau d'émission d'un rayonnement laser (6, 8) renfermant un milieu laser qui détermine la longueur d'onde de son rayonnement laser et en ce que lesdits moyens laser comprennent, en outre, des moyens de pompage optique (12, 14, 16) disposés de manière à fournir de l'énergie de pompage auxdits corps ou matériaux (6, 8), pour amener ces derniers à émettre un rayonnement laser.

**4. -** Appareil suivant la revendication 3, caractérisé en ce que lesdits moyens de pompage optique comprennent au moins deux lampes (12, 14) et des moyens (2) qui assurent le couplage optique de chacune desdites lampes, respectivement, à l'un desdits corps ou matériaux (6, 8).

**5. -** Appareil suivant la revendication 4, caractérisé en ce que chaque lampe (12, 14) est couplée optiquement exclusivement à l'un seulement des corps ou matériaux (6, 8) d'émission des rayonnements laser.

**6. -** Appareil suivant la revendication 5, caractérisé en ce que les moyens de pompage optique comprennent, en outre, une troisième lampe (16) couplée optiquement aux deux corps ou matériaux (6, 8) d'émission des rayonnements laser.

**7. -** Appareil suivant l'une quelconque des revendications 1 à 6, caractérisé en ce que les moyens de conduite ou canalisation du rayonnement laser comprennent : une fibre optique (30) qui s'étend entre les sources des rayonnements laser (6, 8) et les moyens d'application du rayonnement laser (32) ; et des moyens de combinaison du rayonnement (26), disposés entre lesdites sources de rayonnements laser (6, 8) et l'entrée de ladite fibre (30), en vue de combiner les rayonnements laser émanant des deux sources, et d'introduire le rayonnement laser combiné dans la fibre optique.

**8. -** Appareil suivant l'une quelconque des revendication 1 à 6, caractérisé en ce que les moyens de conduite ou canalisation des rayonnements laser comprennent deux fibres optiques (42, 44), qui s'étendent chacune entre les sources de rayonnement laser (6, 8) et les moyens d'application du rayonnement (46) ; et des moyens d'orientation du rayonnement (36, 38, 40), prévus entre lesdites sources de rayonnement et lesdites fibres, en vue de diriger le rayonnement provenant de chaque source jusqu'à l'entrée de l'une des fibres optiques respectives.

**9. -** Appareil suivant l'une quelconque des revendications 1 à 8, caractérisé en ce que la première source de rayonnement produit un rayonnement laser à une longueur d'onde de l'ordre de 0,532µ à 1,5µ , et la seconde source de rayonnement produit un rayonnement laser à une longueur d'onde de l'ordre de 2 à 3µ .

**10. -** Appareil suivant l'une quelconque des revendications 1 à 9, caractérisé en ce que les moyens d'alimentation en fluide de refroidissement sont conçus pour diriger un mélange d'air et d'eau sous forme de brouillard sur le tissu corporel.

**11. -** Appareil selon l'une quelconque des revendications 1 à 10, caractérisé en ce que les sources de rayonnement laser (6, 8) sont constituées par un laser YAG:Nd et par un laser YAG:Er, ou par un laser à alexandrite et par un laser YAG:Er.

**12. -** Appareil suivant l'une quelconque des revendications 4 à 11, caractérisé en ce que les lampes (12, 14, 16) constituant les moyens de pompage optique, contiennent un mélange de Krypton et de xénon.
